(19) Europäisches Patentamt
European Patent Office
Office européen des brevets

(11) EP 0 739 882 B1

(12) **EUROPEAN PATENT SPECIFICATION**

(45) Date of publication and mention of the grant of the patent:
**13.06.2001 Bulletin 2001/24**

(51) Int Cl.[7]: **C07D 221/22**, A61K 31/435

(21) Application number: **96110301.7**

(22) Date of filing: **27.09.1991**

# (54) Novel aconitine compounds and analgesic/anti-inflammatory agent containing the same

Aconitinverbindungen und analgetische/entzündungshemmende Mittel die sie enthalten

Dérivés d'aconitine et agents analgésiques/antiinflammatoires les contenant

(84) Designated Contracting States:
**CH DE FR GB IT LI**

(43) Date of publication of application:
**30.10.1996 Bulletin 1996/44**

(62) Document number(s) of the earlier application(s) in accordance with Art. 76 EPC:
**91917021.7 / 0 564 648**

(73) Proprietor: **SANWA SHOYAKU KABUSHIKI KAISHA**
**Utsunomiya-shi, Tochigi 321 (JP)**

(72) Inventors:
- **Murayama, Mitsuo**
  **Utsunomiya-shi, Tochigi-ken, 320 (JP)**
- **Mori, Takao, c/o Sanwa Shoyaku K.K.**
  **Tochigi-ken, 321 (JP)**

(74) Representative: **Vossius, Volker, Dr. et al**
**Dr. Volker Vossius,**
**Patentanwaltskanzlei - Rechtsanwaltskanzlei,**
**Holbeinstrasse 5**
**81679 München (DE)**

(56) References cited:

| | |
|---|---|
| **FR-A- 1 357 611** | **JP-A- 1 034 965** |
| **JP-A- 1 254 625** | **JP-A- 56 120 620** |
| **JP-A- 63 211 268** | **JP-A- 63 275 583** |

- **CHEMICAL ABSTRACTS, vol. 111, no. 9, 28 August 1989 Columbus, Ohio, US; abstract no. 70963a, page 82; XP002011257 & JP-A-63 211 269 (SANWA SEIYAKU CO LTD) 2 September 1988**
- **CHEMICAL ABSTRACTS, vol. 113, no. 15, 8 October 1990 Columbus, Ohio, US; abstract no. 132566a, page 691; XP002011258 & JP-A-02 076 856 (SANWA SEIYAKU CO LTD) 16 March 1990**
- **CHEMICAL ABSTRACTS, vol. 112, no. 3, 15 January 1990 Columbus, Ohio, US; abstract no. 21188p, page 526; XP002011259 & JP-A-01 143 859 (SANWA SEIYAKU CO LTD) 6 June 1989**
- **CHEMICAL AND PHARMACEUTICAL BULLETIN, vol. 39, no. 2, February 1991, TOKYO JP, pages 379--383, XP000578036 TAKAO MORI ET AL: "Studies on the constituents of Aconitum species. XII. Syntheses of jesaconitine derivatives and their analgesic and toxic activities"**
- **CHEMICAL ABSTRACTS, vol. 110, no. 13, 27 March 1989 Columbus, Ohio, US; abstract no. 111666f, WANG HONGCHENG ET AL: "Studies on the alkaloids from Aconnitum polyschistum Hand-Mazz" page 409; XP002011260 & HETEROCYCLES, vol. 27, no. 7, 1988, pages 1615-1621,**
- **CHEMICAL ABSTRACTS, vol. 116, no. 11, 16 March 1992 Columbus, Ohio, US; abstract no. 106574u, page 808; XP002011261 & JP-A-03 223 255 (SANWA SEIYAKU CO LTD) 2 October 1991**
- **: "", CHEM. PHARM. BULL, , 1991, vol. 39, no. 2, pages 379 to 383**
- **: "", HETEROCYCLES, , 1988, vol. 27, no. 7, pages 1615 to 1621**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 739 882 B1

Printed by Jouve, 75001 PARIS (FR)

## Description

This invention relates to novel aconitine compounds as well as to an analgesic/anti-inflammatory agent containing the same.

Aconitine alkaloids contained in the tuberous root of plants of the genus Aconitium have already been reported to have a potent analgesic and anti-inflammatory action. They, however, are supposed to show a narrow safety margin because of their high toxicity.

As a result of extensive studies in an attempt to develop novel aconitine alkaloid derivatives which are low in toxicity but maintain the analgesic/anti-inflammatory activity that aconitine alkaloids have, we have now succeeded in providing novel compounds of the general formula (I) according to the present invention:

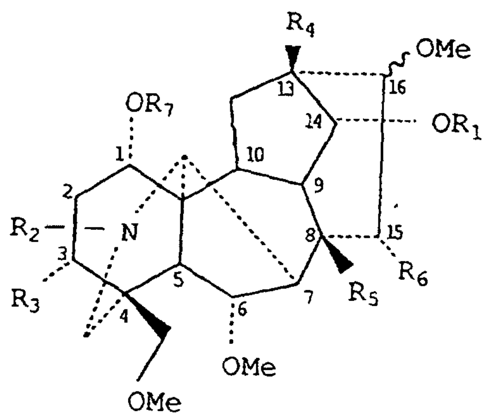

(I)

wherein

(1) $R_1$ is m-chlorobenzoyl, $R_2$ is ethyl, $R_3$ and $R_5$ are hydrogen atoms, $R_4$ is hydroxy, $R_6$ is a group forming a carbonyl group together with the carbon atom at the position 15, and $R_7$ is methyl;
(2) $R_1$ is m-chlorobenzoyl, $R_2$ is ethyl, $R_3$ is a hydrogen atom, $R_4$, $R_5$ and $R_6$ are hydroxy, and $R_7$ is methyl;
(3) $R_1$ is m-chlorobenzoyl, $R_2$ is ethyl, $R_3$ and $R_5$ are hydrogen atoms, $R_4$ and $R_6$ are hydroxy, and $R_7$ is methyl;
(4) $R_1$ is m-chlorobenzoyl, $R_2$ is ethyl, $R_3$ and $R_4$ are hydrogen atoms, $R_5$ and $R_6$ are hydroxy, and $R_7$ is methyl;
(5) $R_1$ is m-chlorobenzoyl, $R_2$ is ethyl, $R_3$, $R_4$ and $R_5$ are hydrogen atoms, $R_6$ is a group forming a carbonyl group together with the carbon atom at the position 15, and $R_7$ is methyl;
(6) $R_1$ is m-chlorobenzoyl, $R_2$ is ethyl, $R_3$, $R_4$ and $R_5$ are hydrogen atoms, $R_6$ is hydroxy, and $R_7$ is methyl;
(7) $R_1$ is p-fluorobenzoyl, m-chlorobenzoyl or p-bromobenzoyl, $R_2$ is ethyl, $R_3$, $R_4$, $R_5$ and $R_6$ are hydroxy, and $R_7$ is methyl;
(8) $R_1$ is p-chlorobenzoyl, $R_2$ is methyl, $R_3$, $R_4$, $R_5$ and $R_6$ are hydroxy, and $R_7$ is methyl;
(9) $R_1$ is m-chlorobenzoyl, $R_2$ is ethyl, $R_3$ and $R_4$ are hydroxy, $R_5$ is a hydrogen atom, $R_6$ is a group forming a carbonyl group together with the carbon atom at the position 15, and $R_7$ is methyl.

We have also found that the compounds of the general formula (I) have a potent analgesic/anti-inflammatory action as well as a lower toxicity than mesaconitine, aconitine, hypaconitine and jesaconitine.

The present invention has been accomplished based on these findings. Thus, the present invention provides novel compounds of the general formula (I) mentioned above as well as an analgesic/anti-inflammatory agent containing as active ingredient one or more of the compounds of the general formula (I) or salts thereof.

The compounds according to the invention of the general formula (I) shown above may be prepared from aconitine alkaloids described in the literature such as aconitine of the formula (II), mesaconitine of the formula (III), hypaconitine of the formula (IV), jesaconitine of the formula (V), 14-O-benzoylmesaconine of the formula (VI), 14-O-benzoylaconine of the formula (VII), 14-O-benzoylhypaconine of the formula (VIII), 14-O-anisoylaconine of the formula (IX) or pyroaconitines or 16-epipyroaconitines of the formula (X) shown below as starting material by way of deoxygenation reactions at the position 3, 8 or 13, replacement of substituents attached to the nitrogen atom or the position 1, 8 or 14 by other substituents, or combination thereof.

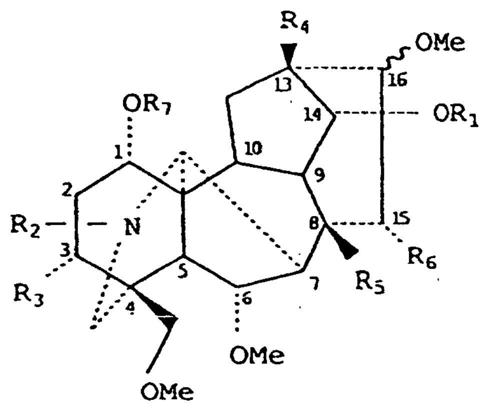

(II) ~ (IX)

In formulas (II) - (IX), $R_1$, $R_2$, $R_3$, $R_4$, $R_5$, $R_6$ and $R_7$ are defined in each case to mean the following:

| | $R_1$ | $R_2$ | $R_3$ | $R_4$ | $R_5$ | $R_6$ | $R_7$ |
|---|---|---|---|---|---|---|---|
| II | Bz | $CH_2CH_3$ | OH | OH | OAc | OH | $CH_3$ |
| III | Bz | $CH_3$ | OH | OH | OAc | OH | $CH_3$ |
| IV | Bz | $CH_3$ | H | OH | OAc | OH | $CH_3$ |
| V | An | $CH_2CH_3$ | OH | OH | OAc | OH | $CH_3$ |
| VI | Bz | $CH_3$ | OH | OH | OH | OH | $CH_3$ |
| VII | Bz | $CH_2CH_3$ | OH | OH | OH | OH | $CH_3$ |
| VIII | Bz | $CH_3$ | H | OH | OH | OH | $CH_3$ |
| IX | An | $CH_2CH_3$ | OH | OH | OH | OH | $CH_3$ |
| Bz:$COC_6H_5$, An:$COC_6H_4OCH_3$, Ac:$COCH_3$ | | | | | | | |

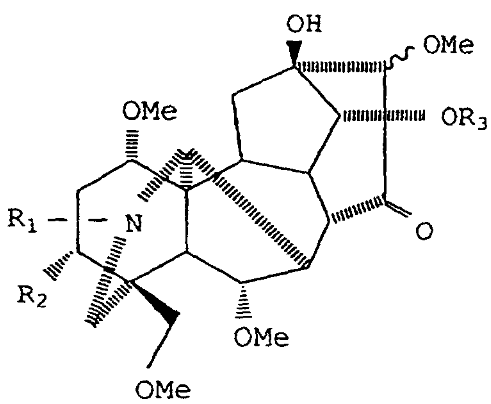

(X)

(wherein $R_1$ is $CH_3$ or $C_2H_5$, $R_2$ is OH or H and $R_3$ is Bz or An.)

The deoxygenation reaction mentioned above may be carried out by using ionic reactions, radical reactions, reduction reactions or the like or a combination thereof, which is normally used in chemical synthesis.

The ionic reaction mentioned above may be carried out by dissolving the corresponding compound in a dehydrating agent as solvent such as sulfuric acid or thionyl chloride, followed by heating under reflux. It may also be carried out by reacting the corresponding compound, while stirring or heating under reflux, with carbon disulfide and methyl iodide in an appropriate solvent such as tetrahydrofuran in the presence, for example, of imidazole and sodium hydride at an appropriate temperature to give the thioacylated product, and adding tributyl-tin hydride to the obtained product in a solvent such as benzene followed by stirring either at room temperature or while heating under reflux.

The radical reaction mentioned above may be carried out by dissolving the corresponding compound in a solvent such as water and phosphoric acid hexamethyltriamide, and irradiating the solution with the use of a low pressure mercury lamp either at room temperature or while cooling.

It may also be carried out by converting the hydroxyl group of the corresponding compound into an active substituent

with the use of oxalyl chloride; adding a radical producing reagent such as α,α'-azoisobutyronitrile and a reducing agent such as tributyl-tin hydride using a solvent such as benzene; and heating the mixture under reflux, or stirring it at room temperature.

The reduction reaction mentioned above may be carried out by subjecting the corresponding compound dissolved in a solvent such as ethanol or acetic acid to contact hydrogenation, either at room temperature or under heating, using as catalyst platinum oxide, palladium/carbon, Raney nickel.

The deoxygenation reaction may also be carried out by subjecting the corresponding compound to conversion of the hydroxyl group into a ketone with an oxidizing agent such as sodium perchromate, followed by derivatization of the ketone into a thioketal or a hydrazone with ethane dithiol, p-toluenesulfonylhydrazine, and then reducing the derivative either at room temperature or under heating or cooling, with an organometallic reducing agent such as lithium aluminum hydride in a solvent such as ether or tetrahydrofuran.

Replacement of a substituent attached to the nitrogen atom with another substituent may be carried out by reacting the corresponding compound with an appropriate oxidizing agent such as potassium permanganate in an appropriate solvent such as acetone to afford the N-dealkylated product, and reacting the resultant N-dealkylated product, for example, with an alkylating or acylating agent such as an acid chloride, alkyl halide or aralkyl halide.

Acylation or alkylation of a hydroxyl group may be carried out by reacting the corresponding compound with an acid chloride, a halogen compound, which is conventionally used for esterification or etherification of a hydroxyl group, in an appropriate solvent such as pyridine.

Replacement of an acyl group present in the form of an ester bond with another acyl group or with an alkyl group may be carried out by deesterification into a hydroxyl group by hydrolysis such as alkaline hydrolysis, followed by acylation or alkylation of the hydroxyl group.

The various reactions mentioned above may be appropriately combined to afford the compounds of formula (I).

The following examples show the preparation of compounds of formula (I). Physicochemical parameters and analytical data of the compounds prepared in these examples are given just after the description of the examples. Pharmacological activity, toxicity of the compounds are shown in Tables 1 - 3 mentioned below.

Example 1

1) 3,13-Dideoxyjesaconitine 100 mg was heated at 180 °C for 30 minutes under reduced pressure (1 - 2 mmHg). The reaction products were subjected to column chromatography on silica gel for separation and purification (eluting solvent : 5 % methanol / chloroform) to give 3,13-dideoxypyrojesaconitine (62 mg).

2) At -70 °C, a tetrahydrofuran (THF) solution containing 3,13-dideoxypyrojesaconitine 60 mg was added in a tetrahydrofuran (THF) solution of $LiAl(OCH_3)_3H$ prepared from 72 mg of $LiAlH_4$ and 0.23 ml of methanol. After this solution was stirred for 1 hour at -70 °C, stirring was further continued for about an additional 1 hour at room temperature. After reaction, wet-THF was added to the reaction mixture at 0 °C and the solid product was filtered off. The filtrate was concentrated to dryness under reduced pressure to give 3,8,13-trideoxyacanine (48 mg).

3) 3,8,13-Trideoxyaconine 30 mg was dissolved in 1 ml of pyridine. To this solution, 30 mg of m-chloro benzoylchloride was added and the mixture vas stirred at 80 °C for 4 hours. After reaction, pyridine was distilled off under reduced pressure. To the residue, water was added and the mixture was made alkaline with 5 % sodium bicarbonate. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 3,8,13-trideoxy-14-O-m-chlorobenzoylaconine (20 mg).

Example 2

1) 3-Deoxyjesaconitine 100 mg was heated at 180 °C for 30 minutes under reduced pressure (1 - 2 mmHg). The reaction products were subjected to column chromatography on silica gel for separation and purification (eluting solvent : 5 % methanol / chloroform) to give 3-deoxypyrojesaconitine (60 mg).

Under the condition of -70 °C, the tetrahydrofuran (THF) solution containing 3-deoxypyrojesaconitine 60 mg was added in the tetrahydrofuran (THF) solution of $LiAl(OCH_3)_2H$ prepared from 7.2 mg of $LiAlH_4$ and 0.23ml of methanol. After this solution was stirred for 1 hour at -70 °C, stirring was continued for about an additional 1 hour at room temperature. After reaction, wet-THF was added to the reaction mixture at 0 °C and the solid product was filtered off. The filtrate was concentrated to dryness under reduced pressure to give 3,8-dideoxyaconine (45.6 mg).

3,8-dideoxyaconine 45 mg were dissolved in a mixture of 5 ml of pyridine and 1 ml of methylene chloride, followed by adding 15 μl of p-anisoyl chloride at -70 °C. The reaction mixture was stirred at the rising temperature from

-70 to -5 °C, which took 2 hours. Then, ice water was added to the reaction mixture and it was made alkaline with 5 % sodium bicarbonate solution. This alkaline solution was extraeted three times with 30 ml of chloroform. The chloroform layer put together was washed with water, dried over anhydrous sodium sulfate and concentrated to dryness under reduced pressure. The residue was subjected to column chromatography on silica gel for separation and purification (eluting solvent : 5 % methanol / ammonia-saturated chloroform) to yield 3,8-dideoxy-14-O-anisoylaconine (32 mg).

2) -ii 3,18-Dideoxy-14-O-anisoylaconine 140 mg was dissolved in 5 ml of methanol. To this solution, 1 ml of 5 % potassium hydroxid was added and the mixture was heated under reflux for 3 hours. After methanol in the reaction mixture was distilled off under reduced pressure, 5 ml of water was added to the residue and the aqueous solution was extracted 3 times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 3,18-dideoxyaconine (62 mg).

3) 3,8-Dideoxyaconine 50 mg was dissolved in 1 ml of pyridine. To this solution, 28 mg of m-chlorobenzoyl chloride was added and the mixture was stirred at 80 °C for 4 hours. After reaction, pyridine was distilled off under reduced pressure. To the residue, water was added and it was made alkaline with 5 % aqueous sodium bicarbonate solution. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 3,8-dideoxy-14-O-m-chlorobenzoyl a conine (23 mg).

Example 3

1) Aconitine 70 mg was dissolved in 5 ml of methanol. To this solution, 1 ml of 5 % potassium hydroxide was added and the mixture was heated under reflux for 3 hours. After methanol in the reaction mixture was distilled off under reduced pressure, 5 ml of water was added to the residue and the aqueous solution was extracted 3 times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give aconine (32 mg).

2) Aconine 30 mg was dissolved in 1 ml of pyridine. To this solution, 30 mg of m-chlorobenzoyl chloride was added and the mixture was stirred at 80 °C for 4 hours. After reaction, pyridine was distilled off under reduced pressure. To the residue, water was added and the mixture was made alkaline with 5 % sodium bicarbonate. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 14-O-m-chlorobenzoyl-aconine (21 mg).

Example 4

1) 13 Dideoxyjesaconitine 30 mg was dissolved in a mixture of dioxane (2 ml) and water (2 ml) and refluxed for 4 hours. After cooling, the solvent was distilled off under reduced pressure. The residue was subjected to thin-layer chromatography for separation and purification (eluting solvent : 5 % methanol / ammonia-saturated chloroform) to give 3,13-dideoxy-14-O-anisoylaconine (22 mg).

2) 3,13-Dideoxy-14-anisoylaconine 70 mg was dissolved in 5 ml of methanol. To this solution, 1 ml of 5 % potassium hydroxide was added and the mixture was heated under reflux for 3 hours. After methanol in the reaction mixture was distilled off under reduced pressure, 5 ml of water was added to the residue and the aqueous solution was extracted 3 times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 3,13-dideoxyaconine (30 mg).

3) 3,13-Dideoxyaconine 30 mg was dissolved in 1 ml of pyridine. To this solution, 30 mg of m-chlorobenzoyl chloride was added and the mixture was stirred at 80 °C for 4 hours. After reaction, pyridine was distilled off under reduced pressure. To the residue, water was added and the reaction mixture was made alkaline with 5 % aqueous sodium bicarbonate solution and extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 3,13-dideoxy-14-O-m-chlorobenzoylaconine (15 mg).

Example 5

1) 3-Deoxyjesaconitine 50 mg was dissolved in a mixture of dioxane (2 ml) and water (2 ml) and refluxed for 4 hours. After cooling, the solvent was distilled off under reduced pressure. The residue was subjected to thin-layer chromatography for separation and purification (eluting solvent : 5 % methanol / ammonia-saturated chloroform) to give 3-deoxy-14-O-anisoylaconine (38mg).
2) 3-deoxy-14-O-anisoylaconine 70 mg was dissolved in 5 ml of methanol. To this solution, 1 ml of 5 % potassium hydroxide was added and the mixture was heated under reflux for 3 hours. After methanol in the reaction mixture was distilled off under reduced pressure, 5 ml of water was added to the residue and the aqueous solution was extracted 3 times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 3-deoxyaconine (35 mg).
3) 3-deoxyaconine 30 mg was dissolved in 1 ml of pyridine. To this solution, 30 mg of m-chlorobenzoyl chloride was added and the mixture was stirred at 80 °C for 4 hours. After reaction, pyridine was distilled off under reduced pressure. To the residue, water was added and the reaction mixture was made alkaline with 5 % aqueous sodium bicarbonate solution and extracted with chloroform. The chloroform layer was hed with water, dried over anhydrous sodium sulfate and concentrated to dryness under reduced pressure. The residue was subjected to column chromatography on silica gel for separation and purification (ammonia-saturated ether) to give 3-deoxy-14-O-m-chlorobenzoyl aconine (17 mg).

Example 6

1) Pyrojesaconitine 70 mg was dissolved in 5 ml of 90 % methanol. To this solution, 15 mg of potassium carbonate was added, and the mixture was stirred at room temperature for 40 hours. After methanol in the reaction mixture was distilled off, 5 ml of water was added and it was extracted three times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 16-epi-pyroaconine (30 mg).
2) 16-Epi-pyroaconine 30 mg was dissolved in 1 ml of pyridine. To this solution, 20 mg of m-chlorobenzoyl chloride was added and the mixture was stirred at 80 °C for 4 hours. After reaction, pyridine was distilled off under reduced pressure and water was added to the residue. After that the aqueous solution was made alkaline with 5 % aqueous sodium bicarbonate. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 14-O-m-chlorobenzoyl-16-epi-pyroaconine (12 mg).

Example 7

1) Pyrojesaconitine 100 mg was dissolved in 1.5 ml of thionyl chloride and refluxed for 3 hours. After cooling, thionyl chloride was distilled off under reduced pressure. To the residue, ice water was added. After this solution was made alkaline with 5 % sodium bicarbonate, it was extracted three times with 30 ml of chloroform. The chloroform layer was washed with water, dried over anhydrous sodium sulfate and concentrated to dryness under reduced pressure. The residue was subjected to column chromatography on silica gel for separation and purification (eluting solvent : ammonia-saturated ether / n-hexane = 4 : 1) to obtain anhydropyrojesaconitine (72.3 mg).

Subsequently, anhydropyrojesaconitine 70 mg was dissolved in 10 ml of ethanol. To this solution, platinic oxide 33 mg was added and it was stirred vigorously in a hydrogen stream for 1.5 hours. After reaction, the catalyst was filtered off and the filtrate was concentrated to dryness under reduced pressure. The residue was subjected to

column chromatography on silica gel for separation and purification (eluting solvent : ammonia-saturated ether / n-hexane = 4 : 1) to give 3-deoxypyrojesaconitine (35 mg).

2) 3-Deoxypyrojesaconitine 35 mg was dissolved in 3 ml of 90 % methanol. To this solution, 10 mg of potassium carbonate was added, and the mixture was stirred at room temperature for 40 hours. After methanol in the reaction mixture was distilled off, 5 ml of water was added to the residue and the mixture was extracted three times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 3-deoxy-16-epi-pyroaconine (20 mg).

3) 3-Deoxy-16-epi-pyroaconine 20 mg was dissolved in 1 ml of pyridine. To this solution, 20 mg of m-chlorobenzoyl chloride was added and the mixture was stirred at 80 °C for 4 hours. After reaction, pyridine was distilled off under reduced pressure. To the residue, water was added and the mixture was made alkaline with 5 % aqueous sodium bicarbonate solution. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 3-deoxy-14-O-m-chlorobenzoyl-16-epi-pyroaconine (10 mg).

Example 8

1) Pyrojesaconitine 100 mg was dissolved in 10 ml of pyridine. To this solution, 0.5 ml of trifluoromethanesulfonic anhydride was added at 0 °C and the mixture was stirred at room temperature for 3 hours. After reaction, the reaction mixture was poured into ice water. After that the solution was made alkaline with ammonia and it was extracted three times with 50 ml of chloroform. The chloroform layer was washed with water, dried over anhydrous sodium sulfate and concentrated to dryness under reduced pressure. The residue was subjected to column chromatography on silica gel for separation and purification (eluting solvent : ammonia-saturated ether / n-hexane = 4 : 1) to give 13-O-trifluoromethanesulfonyl-anhydropyrojesaconitine (98 mg).

Secondly, 13-O-trifluoromethanesulfonyl-anhydropyrojesaconitine 90 mg was dissolved in 100 ml of a mixture of hexamethylphosphoric triamide / water (95 : 5), and irradiated by a low-pressure mercury lamp (2537 Å) in a nitrogen stream at 0 °C for 1 hour. After reaction, the reaction mixture was quenched with five times the volume of water and made alkaline with ammonia. The alkaline solution was extracted three times with 500 ml of ether. The ether layer was washed with water, dried over anhydrous sodium sulfate and concentrated to dryness under reduced pressure. The residue was subjected to column chromatography on silica gel for separation and purification (eluting solvent : ammonia-saturated ether / n-hexane = 4 : 1) to give 13-deoxyanhydropyrojesaconitine (41 mg).

Next, 13-deoxyanhydropyrojesaconitine 40 mg was dissolved in 7 ml of ethanol. To this solution, platinic oxide 15 mg was added and the mixture was stirred vigorously in a hydrogen stream for 1.5 hours. After reaction, the catalyst was filtered off and the filtrate was concentrated to dryness under reduced pressure. The residue was subjected to column chromatography on silica gel for separation and purification (eluting solvent : ammonia-saturated ether / n-hexane = 4 : 1) to give 3,13-dideoxypyrojesaconitine (35 mg).

2) 3,13-Dideoxypyrojesaconitine 35 mg was dissolved in 3 ml of 90 % methanol. To this solution, 10 mg of potassium carbonate was added, and the mixture was stirred at room temperature for 40 hours. After methanol in the reaction mixture was distilled off, 5 ml of water was added to the residue and the mixture was extracted three times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give 3,13-dideoxy-16-epi-pyroaconine (20 mg).

3) 3,13-Dideoxy-16-epi-pyroaconine 20 mg was dissolved in 1 ml of pyridine. To this solution, 20 mg of m-chlorobenzoyl chloride was added and the mixture was stirred at 80 °C for 4 hours. After reaction, pyridine was distilled off under reduced pressure. To the residue, water was added and the mixture was made alkaline with 5 % aqueous sodium bicarbonate solution. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 3,13-dideoxy-14-O-m-chlorobenzoyl-16-epi-pyroaconine (10 mg).

Example 9

1) Mesaconitine 70 mg was dissolved in 5 ml of methanol. To this solution, 1 ml of 5 % potassium hydroxide was added, and the mixture was heated under reflux for 3 hours. After methanol in the reaction mixture was distilled off, 5 ml of water was added to the residue and the mixture was extracted three times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give mesaconine (30 mg).
2) Mesaconine 30 mg was dissolved in 1 ml of pyridine. To this solution, 30 mg of p-chlorobenzoyl chloride was added and the mixture was stirred at 80 °C for 4 hours. After reaction, pyridine was distilled off under reduced pressure. To the residue, water was added and the mixture was made alkaline with 5 % sodium bicarbonate. This alkaline solution was extracted with chloroform. The chloroform layer was washed with water, dried over anhydrous sodium sulfate and concentrated to dryness under reduced pressure. The residue was subjected to column chromatography on silica gel for separation and purification (ammonia-saturated ether) to give 14-O-p-chlorobenzoyl mesaconine (20 mg).

1) Aconitine 70 mg was dissolved in 5 ml of methanol. To this solution, 1 ml of 5 % potassium hydroxide was added and the mixture was heated under reflux for 3 hours. After methanol in the reaction mixture was distilled off under reduced pressure, 5 ml of water was added to the residue and the aqueous solution was extracted 3 times with 10 ml of methylene chloride. The extract was dried over anhydrous sodium sulfate and concentrated to dryness under reduced pressure. The residue was subjected to thin-layer chromatography for separation and purification (eluting solvent : 10 % methanol / ammonia-saturated chloroform) to give aconine (32 mg).
2) 14-O-p-Fluorobenzoylaconine 19 mg was obtained in the same manner as in Example 9-2, except for the use of 30 mg of p-fluorobenzoyl chloride as a substitute for 30 mg of m-chlorobenzoyl chloride and except for the use of 30 mg aconine as a substitute for 30 mg of mesaconine in Example 9-2).

Example 1

14-O-p-Bromobenzoylaconine 20 mg was obtained in the same manner as in Example 10, except for the use of 30 mg of p-bromobenzoyl chloride as a substitute for 30 mg of p-Fluorobenzoyl chloride in Example 10.

(1) Physicochemical parameters and analytical data of 3,8,13-trideoxy-14-O-m-chlorobenzoylaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$(log ε) nm: 231 (4.09), 280 (3.28), 289 (3.25).

4) Analysis of $^1$H-NMR spectra ($CDCl_3$)

The following signals are shown (δ, ppm). 7.78-7.30 (5H, m, m-chlorobenzoyl group), 5.06 (1H, t, J=4.7 Hz, C14-β-H), 3.51, 3.32, 3.28 and 3.25 (each 3H, s, $OCH_3$ at C1, C6, C16 and C18), 1.12 (3H, t, J=7.0 Hz, $CH_3$ of $C_2H_5$ at the nitrogen atom).

5) Analysis of $^{13}$C-NMR spectra ($CDCl_3$)

The following signals are shown (δ, ppm). 165.0 (carbonyl of m-chlorobenzoyl group), 131.6, 130.0, 129.5, 135.0 and 128.4 (m-chlorobenzoyl group), 93.1, 86.0, 85.3, 82.0, 78.3 and 72.5 (C16, C1, C6, C18, C14 and C15),

59.0, 58.7, 58.0 and 56.0 ($OCH_3$ at C18, C16, C6 and Cl), 49.2 ($CH_2$ of $C_2H_5$ at the nitrogen atom), 13.1 ($CH_3$ of $C_2H_5$ at the nitrogen atom).

6) Analysis of Mass spectra

m/z: 589, 591 ($M^+$).

7) Elemental analysis

Calculated for $C_{32}H_{44}NO_7Cl$: C:65.13; H:7.51; N:2,37. Found: C:65.41; H:7.66; N:2.14.

(2) Physicochemical parameters and analytical data of 3,8-dideoxy-14-O-m-chlorobenzoylaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$: 1715 $cm^{-1}$.

3) Analysis of UV spectra (ethanol)

UV $\lambda_{max}^{EtOH}$ (log$\varepsilon$) nm: 231 (4.09), 280 (3.28), 289 (3.25).

4) Analysis of $^1$H-NMR spectra ($CDCl_3$)

The following signals are shown ($\delta$, ppm). 7.98-7.30 (5H, m, m-chlorobenzoyl group), 4.90 (1H, d, J=4.7 Hz, C14-$\beta$-H), 3.70, 3.32, 3.28 and 3.26 (each 3H, s, $OCH_3$ at C1, C6, C16 and C18), 1.11 (3H, t, J=7.0 Hz, $CH_3$ of $C_2H_5$ at the nitrogen atom).

5) Analysis of $^{13}$C-NMR spectra ($CDCl_3$)

The following signals are shown ($\delta$, ppm). 165.0 (carbonyl of m-chlorobenzoyl group), 131.5, 129.9, 129.5, 135.0 and 128.4 (m-chlorobenzoyl group), 94.4, 86.2, 85.0, 82.5, 80.3, 75.3 and 72.6 (C16, C1, C6, C18, C14, C13 and C15), 61.8, 59.1, 58.1 and 56.0 ($OCH_3$ at C16, C18, C6 and C1), 49.1 ($CH_2$ of $C_2H_5$ at the nitrogen atom), 13.2 ($CH_3$ of $C_2H_5$ at the nitrogen atom).

6) Analysis of Mass spectra

m/z: 605, 607 ($M^+$).

7) Elemental analysis

Calculated for $C_{32}H_{44}NO_8Cl$: C: 63.41; H:7.32; N:2.31. Found: C:63.66; H:7.51; N:2.25.

(3) Physicochemical parameters and analytical data of 14-O-m-chlorobenzoylaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$: 1715 $cm^{-1}$.

3) Analysis of UV spectra (ethanol)

UV $\lambda_{max}^{EtOH}$ (logε) nm: 231 (4.09), 280 (3.28), 289 (3.25).

4) Analysis of $^1$H-NMR spectra ($CDCl_3$)

The following signals are shown (δ, ppm). 7.98-7.31 (5H, m, m-chlorobenzoyl group), 4.89 (1H, d, J=5.0 Hz, C14-β-H), 3.71, 3.31, 3.28 and 3.24 (each 3H, s, $OCH_3$ at C1, C6, C16 and C18), 1.02 (3H, t, J=7.0 Hz, $CH_3$ of $C_2H_5$ at the nitrogen atom).

5) Analysis of $^{13}$C-NMR spectra ($CDCl_3$)

166.0 (carbonyl of m-chlorobenzoyl group), 131.5, 130.0, 129.5, 135.1 and 128.3 (m-chlorobenzoyl group), 90.7, 83.4, 82.3, 82.0, 79.4, 78.6, 77.3, 74.7 and 72.0 (C16, C6, C1, C15, C14, C8, C18, C13 and C3), 60.9, 59.1, 57.9 and 55.6 ($OCH_3$ at C16, C8, C6 and C1), 48.8 ($CH_2$ of $C_2H_5$ at the nitrogen atom), 13.0 ($CH_3$ of $C_2H_5$ at the nitrogen atom).

6) Analysis of Mass spectra

m/z: 637, 639 ($M^+$ ).

7) Elemental analysis

Calculated for $C_{32}H_{44}NO_{10}Cl$: C:60.23; H:6.95; N:2.19. Found: C:60.33; H:7.11; N:1.97.

(4) Physicochemical parameters and analytical data of 3,13-dideoxy-14-O-m-chlorobenzoylaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$ (logε) nm: 231 (4.05), 281 (3.25), 289 (3.25).

4) Analysis of $^1$H-NMR spectra ($CDCl_3$)

The following signals are shown (δ, ppm). 7.95-7.28 (5H, m, m-chlorobenzoyl group), 5.01 (1H, t, J=4.9 Hz, C14-β-H), 3.59, 3.30, 3.27 and 3.21 (each 3H, s, $OCH_3$ at C1, C6, C16 and C18), 1.12 (3H, t, J=7.0 Hz, $CH_3$ of $C_2H_5$ at the nitrogen atom).

5) Analysis of $^{13}$C-NMR spectra ($CDCl_3$)

The following signals are shown (δ, ppm). 165.5 (carbonyl of m-chlorobenzoyl group), 131.4, 130.0, 129.5, 135.1 and 128.4 (m-chlorobenzoyl group), 89.6, 85.0, 83.6, 81.8, 80.0, 78.5 and 77.5 (C16, C1, C6, C18, C15, C8 and C14), 59.0, 57.8, 57.6 and 55.6 ($OCH_3$ at C18, C16, C6 and C1), 49.1 ($CH_2$ of $C_2H_5$ at the nitrogen atom), 13.0($CH_3$ of $C_2H_5$ at the nitrogen atom).

6) Analysis of Mass spectra

m/z: 605, 607 ($M^+$).

7) Elemental analysis

Calculated for $C_{32}H_{44}NO_8Cl$: C:63.41; H: 7.32; N: 2.31. Found: C:63.28; H:7.55; N:2.15.

(5) Physicochemical parameters and analytical data of 3-deoxy-14-O-m-chlorobenzoylaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$ (logε) nm: 231 (4.09), 280 (3.28), 289 (3.25).

4) Analysis of $^1$H-MMR spectra ($CDCl_3$)

The following signals are shown (δ, ppm). 7.98-7.30 (5H, m, m-chlorobenzoyl group), 4.87 (1H, d, J=5.0 Hz, C14-β-H), 3.71, 3.31, 3.29 and 3.25 (each 3H, s, $OCH_3$ at C1, C6, C16 and C18), 1.11 (3H, t, J=7.0 Hz, $CH_3$ of $C_2H_5$ at the nitrogen atom).

5) Analysis of $^{13}$C-NMR spectra ($CDCl_3$)

The following signals are shown (δ, ppm). 165.3 (carbonyl of m-chlorobenzoyl group), 131.5, 129.9, 129.4, 135.1 and 128.4 (m-chlorobenzoyl group), 90.7, 85.2, 83.6, 82.0, 81.6, 79.5, 78.6 and 74.8 (C16, C1, C6, C15, C18, C14, C8 and C13), 60.9, 59.2, 58.1 and 55.6 ($OCH_3$ at C16, C18, C6 and Cl), 49.1 ($CH_2$ of $C_2H_5$ at the nitrogen atom), 13.0 ($CH_3$ of $C_2H_5$ at the nitrogen atom).

6) Analysis of Mass spectra

m/z: 621, 623 ($M^+$ ).

7) Elemental analysis

Calculated for $C_{32}H_{44}NO_9Cl$: C:61.78; H:7.13; N:2.25. Found: C:61.91; H:7.32;N:2.28.

(6) Physicochemical parameters and analytical data of 16-epi-14-O-m-chlorobenzoylpyroaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$ (log ε) nm: 231 (4.09), 280 (3.28), 289 (3.25).

4) Analysis of $^1$H-NMR spectra ($CDCl_3$)

The following signals are shown (δ, ppm).

7.98-7.30 (5H, m, m-chlorobenzoyl group), 5.40 (1H, d, J=5.0 Hz, C14-β-H), 3.80, 3.30, 3.28 and 3.26 (each 3H, s, $OCH_3$ at C1, C6, C16 and C18), 1.05 (3H, t, J=7.0 Hz, $CH_3$ of $C_2H_5$ at the nitrogen atom).

5) Analysis of $^{13}$C-NMR spectra ($CDCl_3$)

The following signals are shown (δ, ppm). 211.6 (carbonyl group at C15), 165.0 (carbonyl of m-chlorobenzoyl group), 131.6, 129.8, 129.5, 135.0 and 128.4 (m-chlorobenzoyl group), 86.1, 84.1, 83.6, 78.3, 77.4, 76.8 and 71.9 (C16, C6, C1, C14, C13, C18 and C3), 62.3, 59.2, 57.8 and 56.1 ($OCH_3$ at C16, C18, C6 and C1), 49.1 ($CH_2$ of $C_2H_5$ at the nitrogen atom), 13.4 ($CH_3$ of $C_2H_5$ at the nitrogen atom).

6) Analysis of Mass spectra

m/z: 619, 621 ($M^+$).

7) Elemental analysis

Calculated for $C_{32}H_{42}NO_9Cl$: C:61.98; H: 6.83; N: 2.26. Found: C:62.05; H:6.91; N:2.05.

(7) Physicochemical parameters and analytical data of 3-deoxy-16-epi-14-O-m-chlorobenzoyl-pyroaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$ (logε) nm: 231 (4.09), 280 (3.29), 289 (3.22).

4) Analysis of $^1$H-NMR spectra ($CDCl_3$)

The following signals are shown (δ, ppm). 7.97-7.30 (5H, m, m-chlorobenzoyl group), 5.39 (1H, d, J=5.0 Hz, C14-β-H), 3.81, 3.30, 3.27 and 3.25 (each 3H, s, $OCH_3$ at C1, C6, C16 and C18), 1.04 (3H, t, J=7.0 Hz, $CH_3$ of $C_2H_5$ at the nitrogen atom).

5) Analysis of $^{13}$C-NMR spectra ($CDCl_3$)

The following signals are shown (δ, ppm).
211.6 (carbonyl group at C15), 165.1 (carbonyl of m-chlorobenzoyl group), 131.8, 129.7, 129.5, 135.0 and 128.4 (m-chlorobenzoyl group), 86.2, 85.0, 83.7, 80.2, 78.4 and 77.4 (C16, C1, C6, C18, C14 and C13), 62.3, 59.2, 57.8 and 56.0 ($OCH_3$ at C16, C18, C6 and C1), 49.0 ($CH_2$ of $C_2H_5$ at the nitrogen atom), 13.5 ($CH_3$ of $C_2H_5$ at the nitrogen atom).

6) Analysis of Mass spectra

m/z: 603, 605 ($M^+$).

7) Elemental analysis

Calculated for $C_{32}H_{42}NO_8Cl$: C:63.62; H:7.01; N:2.32. Found: C: 63.41; H:6.94; N:2.22.

(8) Physicochemical parameters and analytical data of 3,13-dideoxy-16-epi-14-O-m-chlorobenzoyl-pyroaconine

1) Property and solubility

Colorless and amorphous powder. Soluble in ether, chloroform, benzene, ethanol, methanol, ethylacetate, pyridine and dimethyl sulfoxide. Insoluble in hexane and water.

2) Analysis of IR spectra (KBr)

IR $\nu_{max}^{KBr}$: 1715 $cm^{-1}$.

3) Analysis of UV spectra (ethanol)

UV $\lambda_{max}^{EtOH}$ (log ε) nm: 231 (4.09), 280 (3.29), 289 (3.22).

4) Analysis of $^1H$-NMR spectra ($CDCl_3$)

The following signals are shown (δ, ppm).
7.96-7.35 (5H, m, m-chlorobenzoyl group), 5.50 (1H, t, J=5.0 Hz, C14-β-H), 3.63, 3.30, 3.27 and 3.25 (each 3H, s, $OCH_3$ at C1, C6, C16 and C18), 1.04 (3H, t, J=7.0 Hz, $CH_3$ of $C_2H_5$ at the nitrogen atom).

5) Analysis of $^{13}C$-NMR spectra ($CDCl_3$)

The following signals are shown (δ, ppm). 211.6 (carbonyl group at C15), 165.0 (carbonyl of m-chlorobenzoyl group), 131.8, 129.7, 129.5, 135.0 and 128.4 (m-chlorobenzoyl group), 85.2, 85.0, 83.7, 80.2 and 78.4 (C16, C1, C6, C18 and C14), 61.1, 59.2, 57.8 and 56.0 ($OCH_3$ at C16, C18, C6 and C1), 49.0 ($CH_2$ of $C_2H_5$ at the nitrogen atom), 13.4 ($CH_3$ of $C_2H_5$ at the nitrogen atom).

6) Analysis of Mass spectra

m/z: 587, 589 ($M^+$ ).

7) Elemental analysis

Calculated for $C_{32}H_{42}NO_7Cl$: C:65.35; H:7.20; N:2.38. Found: C:65.51; H:7.25; N:2.09.

(9) Physicochemical parameters and analytical data of 14-O-p-chlorobenzoylmesaconine

1) Solubility

Colorless and amorphous powder. Soluble in ether, chloroform, benzene, ethanol, methanol, acetone, ethylacetate, pyridine and dimethyl sulfoxide. Insoluble in hexane and water.

2) Analysis of IR spectra (KBr)

IR $\nu_{max}^{KBr}$ : 1715 $cm^{-1}$.

3) Analysis of UV spectra (ethanol)

UV $\lambda_{max}^{EtOH}$ (log ε) nm: 230 (3.98).

4) Analysis of $^1H$-NMR spectra ($CDCl_3$)

The following signals are shown (δ, ppm). 8.00 and 7.43 (each 2H, d, J=8.8 Hz, p-chlorobenzoyl group), 4.95 (1H, d, J=5.4 Hz, C14-β-H), 3.70, 3.32, 3.29 and 3.24 (each 3H, s, $OCH_3$ at C1, C6, C16 and C18), 2.38 (3H, s, $CH_3$ at the nitrogen atom).

5) Analysis of $^{13}C$-NMR spectra ($CDCl_3$)

The following signals are shown (δ, ppm).
165.4 (carbonyl of p-chlorobenzoyl group), 140.0, 131.8, 129.1 and 128.4 (p-chlorobenzoyl group), 90.2, 82.9, 81.9, 81.7, 79.4, 78.3, 77.0, 74.4 and 71.8 (C16, C1, C15, C14, C8, C18 and C13), 60.5, 59.3, 58.2 and 55.9 ($OCH_3$ at C16, C18, C6 and C1), 42.7($CH_3$ at the nitrogen atom).

6) Analysis of EI-Mass spectra

m/z: 623, 625 ($M^+$).

(10) Physicochemical parameters and analytical data of 14-O-p-fluorobenzoylaconine

1) Property and solubility

Colorless and odorless powder. Soluble in ether, chloroform, benzene, ethanol, methanol, acetone, ethylacetate, pyridine and dimethyl sulfoxide. Insoluble in hexane and water.

2) Analysis of IR spectra (KBr)

IR $\nu_{max}^{KBr}$: 1725 $cm^{-1}$.

3) Analysis of UV spectra (ethanol)

UV $\lambda_{max}^{EtOH}$ (log ε) nm: 240 (4.00).

4) Analysis of $^{1}H$-NMR spectra ($CDCl_3$)

The following signals are shown (δ, ppm).
8.00 and 7.42 (each 2H, d, J=8.91 Hz, p-fluorobenzoyl group), 5.02 (1H, d, J=5.2 Hz, C14-β-H), 3.76, 3.33, 3.31 and 3.23 (each 3H, s, $OCH_3$ at C1, C6, C16 and C18), 1.15 (3H, t, J=7.2 Hz, $CH_3$ of $C_2H_5$ at the nitrogen atom).

5) Analysis of $^{13}C$-NMR spectra ($CDCl_3$)

The following signals are shown (δ, ppm). 164.8 (carbonyl of p-fluorobenzoyl group), 160.1, 131.0, 126.5 and 124.8 (p-fluorobenzoyl group), 90.5, 82.8, 82.4, 81.6, 79.5, 78.6, 76.9, 74.4 and 71.4 (C16, C6, C1, C15, C14, C8, C18, C13 and C3), 49.0 ($CH_2$ of $C_2H_5$ at the nitrogen atom), 13.1 ($CH_3$ of $C_2H_5$ at the nitrogen atom).

6) Analysis of EI-Mass spectra

m/z: 621 ($M^+$ ).

(11) Physicochemical parameters and analytical data of 14-O-p-bromobenzoylaconine

1) Property and solubility

Colorless and amorphous powder. Soluble in ether, chloroform, benzene, ethanol, methanol, acetone, ethylacetate, pyridine and dimethyl sulfoxide. Insoluble in hexane and water.

2) Analysis of IR spectra (KBr)

IR $\nu_{max}^{KBr}$ : 1723 $cm^{-1}$.

3) Analysis of UV spectra (ethanol)

UV $\lambda_{max}^{EtOH}$ (logε) nm: 240 (4.02).

4) Analysis of $^1$H-NMR spectra ($CDCl_3$)

The following signals are shown (δ, ppm).
8.04 and 7.42 (each 2H, d, J=8.9, p-bromobenzoyl group), 5.04 (1H, d, J=5.4 Hz, C14-β-H), 3.72, 3.30, 3.29 and 3.27 (each 3H, s, $OCH_3$ at C1, C6, C16 and C18), 1.13 (3H, t, J=7.2 Hz, $CH_3$ of $C_2H_5$ at the nitrogen atom).

5) Analysis of $^{13}$C-NMR spectra ($CDCl_3$)

The following signals are shown (δ, ppm).
165.0 (carbonyl of p-bromobenzoyl group), 140.1, 131.4, 129.2 and 128.6 (p-bromobenzoyl group), 90.5, 83.2, 82.5, 81.7, 80.1, 78.4, 77.0, 74.7 and 71.6 (C16, C6, C1, C15, C14, C8, C18, C13 and C3), 59.6, 59.0, 57.8 and 55.6 ($OCH_3$ at C16, C18, C6 and C1), 49.1 ($CH_2$ of $C_2H_5$ at the nitrogen atom), 13.3 ($CH_3$ of $C_2H_5$ at the nitrogen atom).

6) Analysis of EI-Mass spectra

m/z: 681, 683 ($M^+$ ).
Examples of experiments with respect to the pharmacological property and acute toxicity of the compound shown in formula (I) mentioned above are described below.

Experiment example 1 (Analgesic action)

Measurement of analgesic activity by the acetic acid-induced writhing method.

Male mice of the Std:ddY Strain (20 - 25g) were used. All mice were maintained at room temperature of 24 - 25 °C on a 12 hours light and dark cycle and were given food and water ad libitum. Test compound was used in 3 % suspension in gum arabic, 0.7 % acetic acid in 0.9 % physiological saline solution was injected i.p. (10ml / kg) at 30 minutes after test compound was administered s.c. After 10 minutes, the number of writhing movements was counted for a period of 10 minutes. 3 % gum arabic solution in 0.9 % physiological saline solution was used as a negative control. The $ED_{50}$ values were calculated in accordance with the Litchfield-Wilcoxon's method based on judging to be positive in case of half and under the writhing number of negative control group. The results are shown in Table 1. It was demonstrated in Table 1 that the compounds of this invention had a dose-dependent potent analgesic activity.

Experiment example 2 (Antiinflammatory action)

Measurement of the antiinflammatory activity on the carrageenin-induced hind paw edema in mice.

Male mice of Std:ddY strain (20 - 25g) were used. Thirty minutes after the oral administration of test compound, 25 µl of carrageenin (0.5mg / 25 µl) was injected s.c. under the plantar surface of the right hind paw. To the control group, 25 µl of 0.9 % physiological saline solution was injected S.C. under the plantar surface of the left hind paw. The volume of the paw was measured at one hour intervals for 6 hours with a dial gauge calliper. The results were described as a difference in foot pad thickness between the right and left feet. The results are shown in Table 2. As shown in Table 2, it was demonstrated that the compounds of this invention had an inhibitory action against the carrageenin-induced hind paw edema.

Experiment example 3 (Acute toxicity)

Male mice of the Std:ddY Strain (20 - 25g) were used. The $LD_{50}$ values were calculated using the method of Litchfield-Wilcoxon from the mortality during 72 hours after test compound was administered s.c. The results are shown in Table 3. As shown in Table 3, it was demonstrated that the compounds of this invention were found to be lower in toxicity than mesaconitine, aconitine, hypaconitine and jesaconitine.
As mentioned above, it was indicated that the compound shown in formula (I) had lower toxicity than mesaconitine, aconitine, hypaconitine and jesaconitine and had potent analgesic and antiinflammatory activity.
The dose of the compound of the general formula (I) for clinical use as analgesic/anti-inflammatory agent according to the invention is preferably 1-1,000 mg/day for adults. The agent according to the present invention is presented for actual application after formed into any desired dosage form by conventional methods using customarily used carriers or excipients.
Oral preparations such as tablets, powders, granules and capsules may contain conventional excipients such as calcium carbonate, magnesium carbonate, calcium phosphate, corn starch, potato starch, sugar, lactose, talc, magnesium stearate and gum arabic. Tablets may be coated by conventional methods. Oral liquid preparations may be aqueous or oily suspensions, solutions, syrups, elixirs.

For injectable preparations, the compounds of the formula (I) may be used in the form of a salt thereof, and preferably reconstituted upon use. Such preparations may contain different adjuvants such as suspending, stabilizing or dispersing agents. They may contain sterilized distilled water, refined oils such as peanut oil and corn oil, non-aqueous solvents, polyethylene glycol, polypropylene glycol.

Preparations for rectal administration are presented in the form of compositions for suppository and may contain pharmaceutical carriers well known in the art such as polyethylene glycol, lanolin and coconut oil.

Preparations for topical application are presented in the form of compositions for ointment, plaster or poultice and may contain pharmaceutical carriers well known in the art such as vaseline, paraffin, hydrous lanolin, plastibase, kaolin, bentonite, talc, aluminum silicate, propylene glycol, sorbitol, hydrophilic petrolatum, macrogols, wax, resin, purified lanolin, gum, glycerin, gelatin, polyacrylic acid, polyacrylic acid salt, polyvinyl alcohol, polyvinyl pyrrolidone and polyethylene oxide.

Table 1

| Test compound | $ED_{50}$ value, mg/kg (95 % confidence limits) |
|---|---|
| 3-deoxy-14-O-m-chlorobenzoyl-16-epi-pyroaconine | 10.1 (3.51-29.10) |
| 3,13-dideoxy-14-O-m-chlorobenzoyl-16-epi-pyroaconine | 11.0 (3.41-35.46) |
| 14-O-m-chlorobenzoyl-16-epi-pyroaconine | 11.9 (3.76-37.68) |
| 14-O-p-chlorobenzoylmesaconine | 5.85 (2.28-15.02) |
| 14-O-p-fluorobenzoylaconine | 8.26 (2.86-23.88) |
| 14-O-p-bromobenzoylaconine | 6.42 (2.13-19.39) |

Table 2

| Test compound | Dose (mg/kg) | Difference in foot pad thickness between right and left feet ($\times 10^{-2}$ mm) | | | | | | |
|---|---|---|---|---|---|---|---|---|
| | | 0 | 1 | 2 | 3 | 4 | 5 | 6 (h) |
| 3-deoxy-14-O-m-chlorobenzoyl-16-epi-pyroaconine | 10 | 2±1 | 65± 9 | 76± 9 | 75±10* | 74± 7* | 78±12 | 77± 8 |
| | 20 | 1±2 | 53± 8* | 59± 4* | 63± 8* | 68±11* | 62± 4** | 67± 6* |
| 3,13-dideoxy-14-O-m-chlorobenzoyl-16-epi-pyroaconine | 10 | 1±1 | 70± 6 | 82±10 | 91± 7 | 94± 8 | 90±11 | 84± 6 |
| | 20 | 0±1 | 60± 8 | 64± 8 | 61±10* | 64±12* | 70± 4* | 69±13 |

Table 2 (continued)

| Test compound | Dose (mg/kg) | Difference in foot pad thickness between right and left feet (×$10^{-2}$ mm) | | | | | | |
|---|---|---|---|---|---|---|---|---|
| | | 0 | 1 | 2 | 3 | 4 | 5 | 6 (h) |
| 14-O-m-chlorobenzoyl-16-epi-pyroaconine | 10 | -1±1 | 63±14 | 64±11 | 67±12* | 65± 7** | 69± 5 | 66±14 |
| | 20 | 4±2 | 57± 8* | 60±13 | 63± 8** | 62± 9** | 59± 6** | 56± 9* |

Table 2 (continued)

| Test compound | Dose (mg/kg) | Difference in foot pad thickness between right and left feet (×$10^{-2}$ mm) 0 | 1 | 2 | 3 | 4 | 5 | 6 (h) |
|---|---|---|---|---|---|---|---|---|
| 14-O-p-chloro-benzoylmesaconine | 10 | 5±2 | 65± 9 | 74± 5 | 73± 7* | 78± 8* | 81±11 | 85± 9 |
| | 30 | 4±3 | 51±11 | 59± 6* | 56±10** | 64± 6** | 76±10 | 74±10* |
| 14-O-p-fluoro-benzoylaconine | 10 | 1±4 | 67± 8 | 80±10 | 85± 7 | 94± 8 | 89±12 | 88± 9 |
| | 30 | 4±4 | 57±10 | 63± 8 | 61± 6** | 69±10* | 72± 6* | 73±13 |
| 14-O-p-bromo-benzoylaconine | 10 | 2±3 | 60± 8 | 66±10 | 71±13 | 74± 7* | 73±14 | 76± 9 |
| | 30 | 5±2 | 58± 9 | 53± 8* | 59± 9** | 62± 6** | 68± 7** | 71±18 |

Table 3

| Test compound | $LD_{50}$ value, mg/kg (95 % confidence limits) |
|---|---|
| 3-deoxy-14-O-m-chlorobenzoyl-16-epi-pyroaconine | 20< |
| 3,13-dideoxy-14-O-m-chlorobenzoyl-16-epi-pyroaconine | 20< |
| 14-O-m-chlorobenzoyl-16-epi-pyroaconine | 20< |
| 14-O-p-chlorobenzoylmesaconine | 30< |
| 14-O-p-fluorobenzoylaconine | 30< |
| 14-O-p-bromobenzoylaconine | 30< |

## Claims

**1.** An aconitine compound of the formula (I)or a salt thereof:

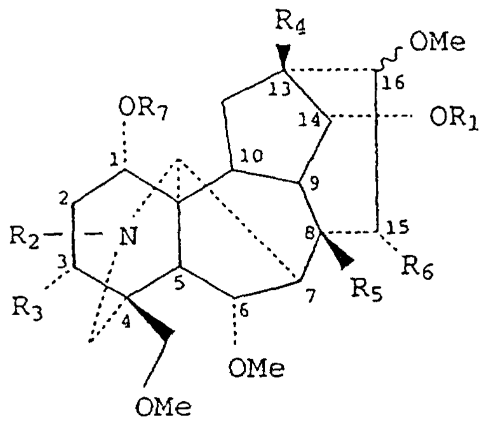

(I)

wherein

(1) $R_1$ is m-chlorobenzoyl, $R_2$ is ethyl, $R_3$ and $R_5$ are hydrogen atoms, $R_4$ is hydroxy, $R_6$ is a group forming a carbonyl group together with the carbon atom at the position 15, and $R_7$ is methyl;
(2) $R_1$ is m-chlorobenzoyl, $R_2$ is ethyl, $R_3$ is a hydrogen atom, $R_4$, $R_5$ and $R_6$ are hydroxy, and $R_7$ is methyl;
(3) $R_1$ is m-chlorobenzoyl, $R_2$ is ethyl, $R_3$ and $R_5$ are hydrogen atoms, $R_4$ and $R_6$ are hydroxy, and $R_7$ is methyl;
(4) $R_1$ is m-chlorobenzoyl, $R_2$ is ethyl, $R_3$ and $R_4$ are hydrogen atoms, $R_5$ and $R_6$ are hydroxy, and $R_7$ is methyl;
(5) $R_1$ is m-chlorobenzoyl, $R_2$ is ethyl, $R_3$, $R_4$ and $R_5$ are hydrogen atoms, $R_6$ is a group forming a carbonyl group together with the carbon atom at the position 15, and $R_7$ is methyl;
(6) $R_1$ is m-chlorobenzoyl, $R_2$ is ethyl, $R_3$, $R_4$ and $R_5$ are hydrogen atoms, $R_6$ is hydroxy, and $R_7$ is methyl;
(7) $R_1$ is p-fluorobenzoyl, m-chlorobenzoyl or p-bromobenzoyl, $R_2$ is ethyl, $R_3$, $R_4$, $R_5$ and $R_6$ are hydroxy, and $R_7$ is methyl;
(8) $R_1$ is p-chlorobenzoyl, $R_2$ is methyl, $R_3$, $R_4$, $R_5$ and $R_6$ are hydroxy, and $R_7$ is methyl;
(9) $R_1$ is m-chlorobenzoyl, $R_2$ is ethyl, $R_3$ and $R_4$ are hydroxy, $R_5$ is a hydrogen atom, $R_6$ is a group forming a carbonyl group together with the carbon atom at the position 15, and $R_7$ is methyl.

**2.** An analgesic/anti-inflammatory composition containing as active ingredient an aconitine compound of claim 1 or a salt thereof together with an excipient.

**3.** The use of an aconitine compound of claim 1 for the preparation of a pharmaceutical composition having analgesic/anti-inflammatory activity.

**Patentansprüche**

1. Eine Aconitinverbindung der allgemeinen Formel

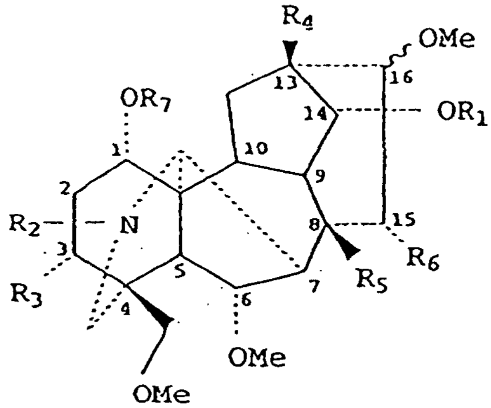

(I)

oder ein Salz davon, worin

(1) $R_1$ eine m-Chlorbenzoylgruppe, $R_2$ eine Ethylgruppe ist, $R_3$ und $R_5$ Wasserstoffatome sind, $R_4$ eine Hydroxylgruppe ist, $R_6$ eine Gruppe ist, die zusammen mit dem Kohlenstoffatom an der Position 15 eine Carbonylgruppe bildet und $R_7$ eine Methylgruppe ist;
(2) $R_1$ eine m-Chlorbenzoylgruppe, $R_2$ eine Ethylgruppe, $R_3$ ein Wasserstoffatom ist, $R_4$, $R_5$ und $R_6$ Hydroxylgruppen sind und $R_7$ eine Methylgruppe ist;
(3) $R_1$ eine m-Chlorbenzoylgruppe, $R_2$ eine Ethylgruppe ist, $R_3$ und $R_5$ Wasserstoffatome sind, $R_4$ und $R_6$ Hydroxylgruppen sind und $R_7$ eine Methylgruppe ist;
(4) $R_1$ eine m-Chlorbenzoylgruppe, $R_2$ eine Ethylgruppe ist, $R_3$ und $R_4$ Wasserstoffatome sind, $R_5$ und $R_6$ Hydroxylgruppen sind und $R_7$ eine Methylgruppe ist;
(5) $R_1$ eine m-Chlorbenzoylgruppe, $R_2$ eine Ethylgruppe ist, $R_3$, $R_4$ und $R_5$ Wasserstoffatome sind, $R_6$ eine Gruppe ist, die zusammen mit dem Kohlenstoffatom an der Position 15 eine Carbonylgruppe bildet und $R_7$ eine Methylgruppe ist;
(6) $R_1$ eine m-Chlorbenzoylgruppe, $R_2$ eine Ethylgruppe ist, $R_3$, $R_4$ und $R_5$ Wasserstoffatome sind, $R_6$ eine Hydroxylgruppe und $R_7$ eine Methylgruppe ist;
(7) $R_1$ eine p-Fluorbenzoyl-, m-Chlorbenzoyl- oder p-Brombenzoylgruppe ist, $R_2$ eine Ethylgruppe ist, $R_3$, $R_4$, $R_5$ und $R_6$ Hydroxylgruppen sind und $R_7$ eine Methylgruppe ist;
(8) $R_1$ eine p-Chlorbenzoylgruppe, $R_2$ eine Methylgruppe ist, $R_3$, $R_4$, $R_5$ und $R_6$ Hydroxylgruppen sind und $R_7$ eine Methylgruppe ist;
(9) $R_1$ eine m-Chlorbenzoylgruppe, $R_2$ eine Ethylgruppe ist, $R_3$ und $R_4$ Hydroxylgruppen sind, $R_5$ ein Wasserstoffatom ist, $R_6$ eine Gruppe ist, die zusammen mit dem Kohlenstoffatom an der Position 15 eine Carbonylgruppe bildet und $R_7$ eine Methylgruppe ist;

2. Eine analgetische/entzündungshemmende Zusammensetzung, die als Wirkstoff eine Aconitinverbindung nach Anspruch 1 oder ein Salz davon zusammen mit einem Träger enthält.

3. Die Verwendung einer Aconitinverbindung nach Anspruch 1 zur Herstellung einer pharmazeutischen Zusammensetzung mit analgetischer/entzündungshemmender Wirkung.

**Revendications**

1. Composé d'aconitine de formule (I) ou sel de celui-ci:

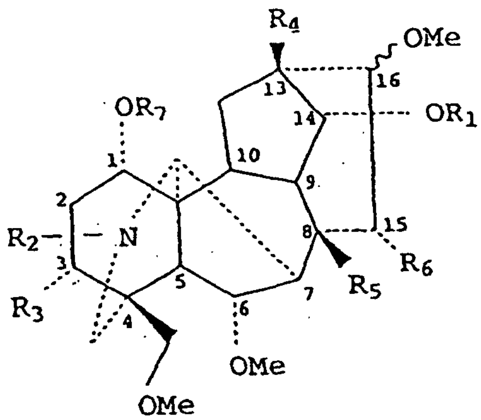

(I)

où

(1) $R_1$ est un m-chlorobenzoyle, $R_2$ est un éthyle, $R_3$ et $R_5$ sont des atomes d'hydrogène, $R_4$ est un hydroxy, $R_6$ est un groupe formant un groupe carbonyle conjointement avec l'atome de carbone sur la position 15, et $R_7$ est un méthyle;

(2) $R_1$ est un m-chlorobenzoyle, $R_2$ est un éthyle, $R_3$ est un atome d'hydrogène, $R_4$, $R_5$ et $R_6$ sont l'hydroxy, et $R_7$ est un méthyle;

(3) $R_1$ est un m-chlorobenzoyle, $R_2$ est un éthyle, $R_3$ et $R_5$ sont des atomes d'hydrogène, $R_4$ et $R_6$ sont l'hydroxy, et $R_7$ est un méthyle;

(4) $R_1$ est un m-chlorobenzoyle, $R_2$ est un éthyle, $R_3$ et $R_4$ sont des atomes d'hydrogène, $R_5$ et $R_6$ sont l'hydroxy, et $R_7$ est un méthyle;

(5) $R_1$ est un m-chlorobenzoyle, $R_2$ est un éthyle, $R_3$, $R_4$ et $R_5$ sont des atomes d'hydrogène, $R_6$ est un groupe formant un groupe carbonyle conjointement avec l'atome de carbone sur la position 15, et $R_7$ est un méthyle;

(6) $R_1$ est un m-chlorobenzoyle, $R_2$ est un éthyle, $R_3$, $R_4$ et $R_5$ sont des atomes d'hydrogène, $R_6$ est un hydroxy, et $R_7$ est un méthyle;

(7) $R_1$ est un p-fluorobenzoyle, un m-chlorobenzoyie ou un p-bromobenzoyle, $R_2$ est un éthyle, $R_3$, $R_4$, $R_5$ et $R_6$ sont l'hydroxy, et $R_7$ est un méthyle;

(8) $R_1$ est un p-chlorobenzoyle, $R_2$ est un méthyle, $R_3$, $R_4$, $R_5$ et $R_6$ sont l'hydroxy, et $R_7$ est un méthyle;

(9) $R_1$ est un m-chlorobenzoyle, $R_2$ est un éthyle, $R_3$ et $R_4$ sont l'hydroxy, $R_5$ est un atome d'hydrogène, $R_6$ est un groupe formant un groupe carbonyle conjointement avec l'atome de carbone sur la position 15, et $R_7$ est un méthyle.

**2.** Composition analgésique/anti-inflammatoire contenant comme composant actif un composé d'aconitine selon la revendication 1 ou un sel de celui-ci, conjointement avec un excipient.

**3.** Utilisation d'un composé d'aconitine selon la revendication 1 pour la préparation d'une composition pharmaceutique ayant une activité analgésique /anti-inflammatoire.